# EUROPEAN PATENT APPLICATION

(11) **EP 4 671 341 A1**
(43) Date of publication of application: **31.12.2025**
(21) Application number: 24780597.1
(22) Date of filing: 28.03.2024
(51) Int. Cl.: C09K 3/00, C07D 249/20, C08G 18/38, G02C 7/00

(54) **ULTRAVIOLET ABSORBER AND SPECTACLE LENS**

(30) Priority: 31.03.2023 JP 2023058603
(71) Applicant: HOYA LENS THAILAND LTD., Pathumthani 12130 (TH)
(72) Inventor: KOUSAKA, Masahisa, Tokyo 160-8347 (JP); OHNISHI, Tomofumi, Tokyo 160-8347 (JP)
(74) Representative: Kraus & Lederer PartGmbB
(86) International application number: PCT/JP2024/012568
(87) International publication number: WO 2024/204496

(57) **Abstract**

An ultraviolet light absorbing agent containing a compound (1) represented by Formula (1) (where, R² is an alkyl group having 1 to 12 carbon atoms or an alkoxy group having 1 to 12 carbon atoms, and n is 0 or 1),
wherein, in a spectrum of the ultraviolet light absorbing agent measured by liquid chromatography using a diode array detector, the peak area ratio of a compound (2) represented by Formula (2) (where, R² is an alkyl group having 1 to 12 carbon atoms or an alkoxy group having 1 to 12 carbon atoms, and n is 0 or 1) is 0.008% or less:

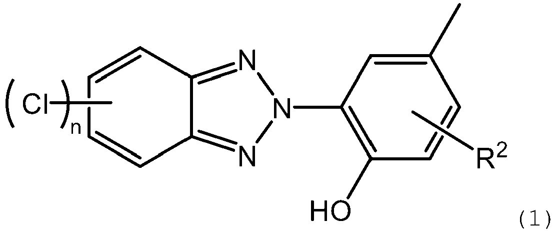

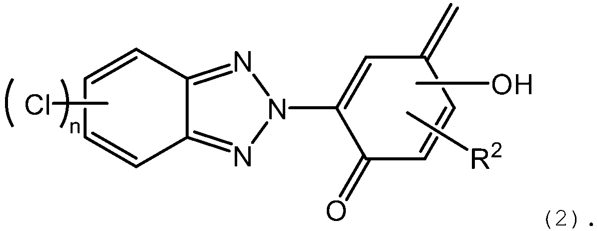

## Description

### [Technical Field]

The present disclosure relates to an ultraviolet light absorbing agent and a spectacle lens.

### [Background Art]

In spectacle lenses, when light beams in a blue region (wavelength range of 380 to 500 nm) are cut, glare is reduced, and visibility and contrast are improved. In addition, for eye health, since light beams in a blue region (380 to 500 nm) have a high energy, they are said to cause damage to the retina and the like. Damage caused by blue light is called "blue light hazard," and it is said that particularly the vicinity of 380 to 420 nm on the low wavelength side is most dangerous, and it is desirable to cut light in this region.

PTL 1 describes an optical material that contains one or more ultraviolet light absorbing agents (a) having a maximum absorption peak in a range of 350 nm or more and 370 nm or less and has a thickness of 2 mm in which the measured light transmittance satisfies the following properties (1) to (3) [(1) the light transmittance at a wavelength of 410 nm is 10% or less, (2) the light transmittance at a wavelength of 420 nm is 70% or less, and (3) the light transmittance at a wavelength of 440 nm is 80% or more].

### [Citation List]

### [Patent Literature]

[PTL 1] WO 2014/133111

### [Summary of Invention]

### [Technical Problem]

According to the conventional spectacle lens as disclosed in PTL 1, when a specific ultraviolet light absorbing agent is contained, it is possible to reduce the transmittance of light with a wavelength of 410 nm. However, generally, when an ultraviolet light absorbing agent exhibits light absorption properties at a wavelength of 410 nm, it is also possible to absorb light in the vicinity of the wavelength. Examples of such ultraviolet light absorbing agents include 2-(3-tert-butyl-2-hydroxy-5-methylphenyl)-5-chloro-2H-benzotriazole, but they have a problem that they deteriorate over time during storage and unnecessarily become yellow.

One embodiment of the present disclosure relates to an ultraviolet light absorbing agent with reduced coloration, and a spectacle lens.

### [Solution to Problem]

One embodiment of the present disclosure relates to an ultraviolet light absorbing agent containing a compound (1) represented by Formula (1): 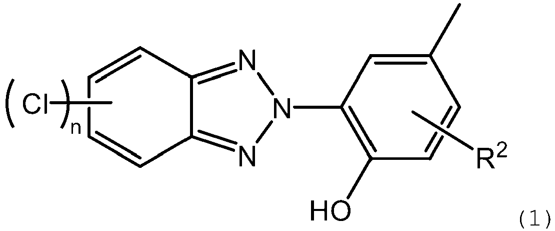 (where,
R² is an alkyl group having 1 to 12 carbon atoms or an alkoxy group having 1 to 12 carbon atoms, and
n is 0 or 1),
wherein, in a spectrum of the ultraviolet light absorbing agent measured by liquid chromatography using a diode array detector, the peak area ratio of a compound (2) represented by Formula (2) is 0.008% or less: 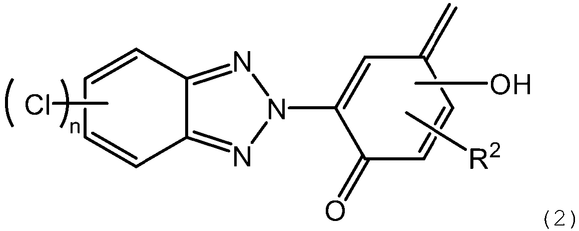 (where,
   R² is an alkyl group having 1 to 12 carbon atoms or an alkoxy group having 1 to 12 carbon atoms, and
   n is 0 or 1).

One embodiment of the present disclosure
relates to a spectacle lens including a lens substrate including the ultraviolet light absorbing agent and a resin.

One embodiment of the present disclosure relates to a method of producing ultraviolet light absorbing agent crystals containing a compound (1) represented by Formula (1): 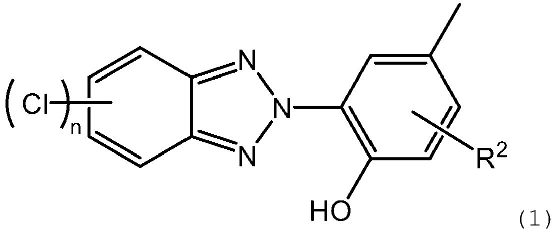 (where,
R² is an alkyl group having 1 to 12 carbon atoms or an alkoxy group having 1 to 12 carbon atoms, and
n is 0 or 1), the method including:
   a step of adding an isopropanol solution to a toluene solution containing the ultraviolet light absorbing agent to obtain crystals; and
   a step of washing the crystals with isopropanol.

### [Advantageous Effects of Invention]

According to one embodiment of the present disclosure, it is possible to provide an ultraviolet light absorbing agent with reduced coloration and a spectacle lens.

### [Brief Description of Drawings]

[Fig. 1]

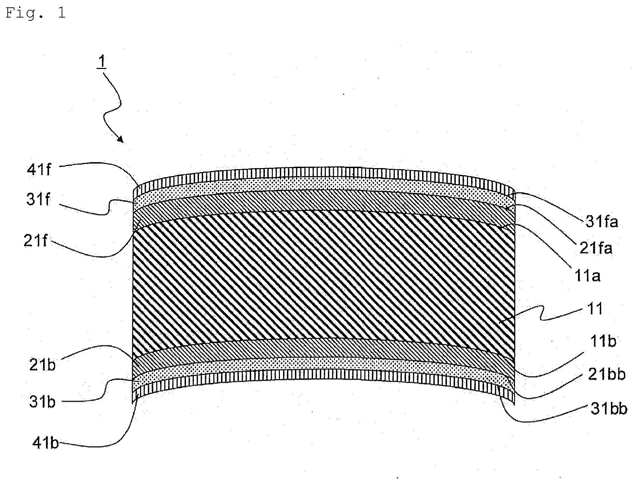

Fig. 1 is a schematic cross-sectional view of a spectacle lens 1 according to the present embodiment.

### [Description of Embodiments]

Hereinafter, embodiments according to the present disclosure will be described in detail with reference to the drawings as necessary, but the present disclosure is not limited thereto, and can be variously modified without departing from the scope and spirit thereof. Here, in the drawings, the same components are denoted with the same reference numerals and redundant descriptions are omitted. In addition, positional relationships such as above, below, left and right are based on the positional relationships shown in the drawings unless otherwise specified. In addition, dimensional ratios in the drawings are not limited to the illustrated ratios.

Here, in this specification, for example, the expression of a numerical value range of "1 to 100" includes both the lower limit value "1" and the upper limit value "100." In addition, the same applies to the expression of other numerical value ranges.

In addition, "a cured product of an isocyanate component and an active hydrogen compound component" does not mean that other components are excluded but means that a cured composition contains at least an isocyanate component and an active hydrogen compound component.

### [Ultraviolet Light Absorbing Agent]

The ultraviolet light absorbing agent according to the present embodiment is an ultraviolet light absorbing agent containing
a compound (1) represented by Formula (1): 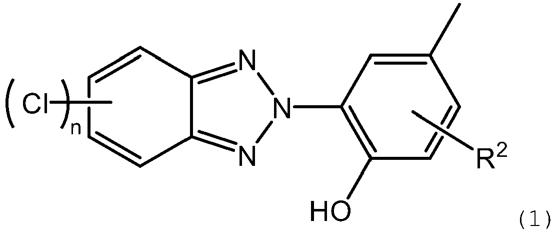 (where,
R² is an alkyl group having 1 to 12 carbon atoms or an alkoxy group having 1 to 12 carbon atoms, and
n is 0 or 1)
wherein, in a spectrum of the ultraviolet light absorbing agent measured by liquid chromatography using a diode array detector, the peak area ratio of a compound (2) represented by Formula (2) is 0.008% or less: 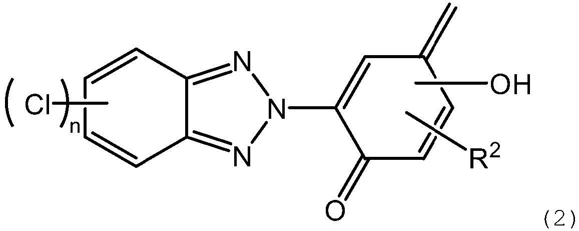 (where,
   R² is an alkyl group having 1 to 12 carbon atoms or an alkoxy group having 1 to 12 carbon atoms, and
   n is 0 or 1).

According to the above ultraviolet light absorbing agent, it is possible to provide an ultraviolet light absorbing agent with reduced coloration and a spectacle lens.

In the formula, R² represents an alkyl group having 1 to 12 carbon atoms or an alkoxy group having 1 to 12 carbon atoms, and the number of carbon atoms of the alkyl group and the number of carbon atoms of the alkoxy group are each independently preferably 1 to 8, more preferably 2 to 8, and still more preferably 4 to 8. The alkyl group and alkoxy group may be branched or linear. Among alkyl groups and alkoxy groups, alkoxy groups are preferable.

Examples of alkyl groups include a methyl group, an ethyl group, an n-propyl group, an isopropyl group, an n-butyl group, a sec-butyl group, a tert-butyl group, a pentyl group, a hexyl group, a heptyl group, an n-octyl group, a 1,1,3,3-tetramethylbutyl group, a nonyl group, a decyl group, an undecyl group, and a dodecyl group.

Examples of alkoxy groups include a methoxy group, an ethoxy group, an n-propyloxy group, an isopropyloxy group, an n-butyloxy group, a sec-butyloxy group, a tert-butyloxy group, a pentyloxy group, a hexyloxy group, a heptyloxy group, an octyloxy group, a nonyloxy group, a decyloxy group, an undecyloxy group, and a dodecyloxy group. Among these, a tert-butyl group is preferable.

m is 0 or 1, and preferably 0.

n is 0 or 1, and preferably 1.

The compound (1) is a compound (1-1) represented by Formula (1-1): 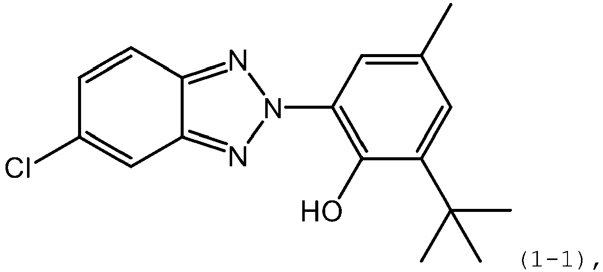 and .
the compound (2) is preferably a compound (2-1) represented by Formula (2-1):

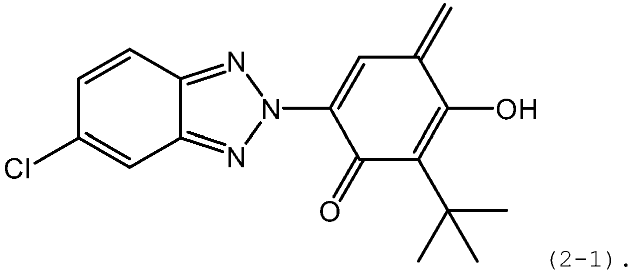

### (Peak Area Ratio)

In a spectrum of the ultraviolet light absorbing agent according to the present embodiment measured by liquid chromatography using a diode array detector, the peak area ratio of the compound (2) represented by Formula (2) is 0.008% or less. By achieving this peak area ratio, coloration of the ultraviolet light absorbing agent is reduced.

For the same reason, the peak area ratio is preferably 0.006% or less, more preferably 0.005% or less, and still more preferably 0.004% or less. The peak area ratio may be, for example, 0.001% or more.

In a spectrum of the ultraviolet light absorbing agent according to the present embodiment measured by liquid chromatography using a diode array detector, the peak area ratio of the compound (1) represented by Formula (1) is preferably 95% or more, more preferably 98% or more, still more preferably 99% or more.

The measurement method by liquid chromatography using a diode array detector is a method described in examples.

In order to achieve the above peak area, the ultraviolet light absorbing agent is purified by repeated recrystallization using toluene and isopropanol.

More specifically, a mother liquor for recrystallization is prepared by adding 40 to 60 vol% of isopropanol with respect to a toluene solvent to a toluene solution containing an ultraviolet light absorbing agent with a concentration of 5 to 10%. The temperature is then lowered to perform recrystallization. Furthermore, the obtained recrystallized product is removed and washed with isopropanol. When these steps are performed, an ultraviolet light absorbing agent having the above peak area ratio is obtained.

### [Spectacle Lens]

The spectacle lens according to the present embodiment includes a lens substrate including the ultraviolet light absorbing agent and a resin. The spectacle lens according to the present embodiment may include at least one layer selected from the group consisting of a hard coat layer, a foundation layer, and an antireflection layer.

Fig. 1 is a schematic cross-sectional view of a spectacle lens 1 according to the present embodiment. The spectacle lens 1 according to the present embodiment includes a lens substrate 11, a hard coat layer 21f provided on the side of an object-side surface 11a of the lens substrate 11, a functional layer 31f provided on the side of an object-side surface 21fa of the hard coat layer 21f, and an antifouling layer 41f provided on the side of an object-side surface 31fa of the functional layer 31f.

In addition, when the lens substrate 11 is a finished lens, the spectacle lens 1 according to the present embodiment further includes a hard coat layer 21b provided on the side of an eyeball-side surface 11b of the lens substrate 11, a functional layer 31b provided on the side of an eyeball-side surface 21bb of the hard coat layer 21b, and a water-repellent layer 41b provided on the side of an eyeball-side surface 31bb of the functional layer 31b.

Here, although not shown, a foundation layer may be provided between the lens substrate 11 and the hard coat layer 21f or between the lens substrate 11 and the hard coat layer 21b.

### <Lens Substrate>

The lens substrate includes the ultraviolet light absorbing agent and a resin.

The spectacle lens preferably contains 0.05 parts by mass or more and 2.00 parts by mass or less of the compound 1 with respect to 100 parts by mass of the resin in the lens substrate. In order to further reduce the transmittance of light with a wavelength of 410 nm, the content of the ultraviolet light absorbing agent with respect to 100 parts by mass of the resin in the lens substrate is preferably 0.10 parts by mass or more and 2.00 parts by mass or less, more preferably 0.15 parts by mass or more and 1.50 parts by mass or less, and still more preferably 0.20 parts by mass or more and 1.00 part by mass or less.

### [Resin]

Examples of resins for lens substrates include urethane resins, episulfide resins, polycarbonate resins, and acrylic resins.

The resin is preferably at least one selected from the group consisting of a polythiourethane resin, a polysulfide resin, and a polyurethane resin, and more preferably at least one selected from the group consisting of a polythiourethane resin and a polysulfide resin.

### (Urethane Resin)

The urethane resin is a cured product of a polymerizable composition containing an isocyanate component and an active hydrogen compound component. Examples of urethane resins include a thiourethane resin having a polymerization moiety of an isocyanate component and a polythiol component; a urethane resin having a polymerization moiety of an isocyanate component and a polyol component; and a urethane urea resin having a polythiourethane moiety which is a polymerization moiety of an isocyanate component and a polythiol component or a polyol component and a polyurea moiety which is a polymer of an isocyanate component and a polyamine component.

### (Isocyanate Component)

Examples of isocyanate components include polyisocyanate compounds having an aromatic ring, polyisocyanate compounds having an aliphatic ring, and linear or branched aliphatic polyisocyanate compounds.

Examples of polyisocyanate compounds having an aromatic ring include diisocyanatobenzene, 2,4-tolylene diisocyanate, 2,6-tolylene diisocyanate, ethyl phenylene diisocyanate, isopropyl phenylene diisocyanate, diethyl phenylene diisocyanate, diethyl phenylene diisocyanate, diisopropyl phenylene diisocyanate, trimethylbenzene triisocyanate, benzene triisocyanate, biphenyl diisocyanate, 4,4'-diphenylmethane diisocyanate, 4,4'-methylenebis(2-methylphenylisocyanate), bibenzyl-4,4'-diisocyanate, bis(isocyanatophenyl)ethylene, 1,3-bis(isocyanatomethyl)benzene, 1,4-bis(isocyanatomethyl)benzene, 1,3-bis(isocyanatoethyl)benzene, bis(isocyanatopropyl)benzene, α,α,α',α'-tetramethylxylylene diisocyanate, bis(isocyanatobutyl)benzene, bis(isocyanatomethyl)naphthalene, bis(isocyanatomethylphenyl)ether, 2-isocyanatophenyl-4-isocyanatophenyl sulfide, bis(4-isocyanatophenyl)sulfide, bis(4-isocyanatomethylphenyl)sulfide, bis(4-isocyanatophenyl)disulfide, bis(2-methyl-5-isocyanatophenyl)disulfide, bis(3-methyl-5-isocyanatophenyl)disulfide, bis(3-methyl-6-isocyanatophenyl)disulfide, bis(4-methyl-5-isocyanatophenyl)disulfide, bis(3-methyloxy-4-isocyanatophenyl)disulfide, and bis(4-methyloxy-3-isocyanatophenyl)disulfide.

Examples of polyisocyanate compounds having an aliphatic ring include 1,3-diisocyanatocyclohexane, 1,4-diisocyanatocyclohexane, isophorone diisocyanate, 1,3-bis(isocyanatomethyl)cyclohexane, 1,4-bis(isocyanatomethyl)cyclohexane, dicyclohexylmethane-4,4'-diisocyanate, dicyclohexylmethane-2,4'-diisocyanate, 2,5-bis(isocyanatomethyl)-bicyclo[2.2.1]heptane, 2,6-bis(isocyanatomethyl)-bicyclo[2.2.1]heptane, 2,5-diisocyanato-1,4-dithiane, 2,5-bis(isocyanatomethyl)-1,4-dithiane, 4,5-diisocyanato-1,3-dithiolane, 4,5-bis(isocyanatomethyl)-1,3-dithiolane, and 4,5-bis(isocyanatomethyl)-2-methyl-1,3-dithiolane.

Examples of linear or branched aliphatic polyisocyanate compounds include pentamethylene diisocyanate, hexamethylene diisocyanate, 2,2-dimethylpentane diisocyanate, 2,2,4-trimethylhexane diisocyanate, butene diisocyanate, 1,3-butadiene-1,4-diisocyanate, 2,4,4-trimethylhexamethylene diisocyanate, 1,6,11-undecane triisocyanate, 1,3,6-hexamethylenetriisocyanate, 1,8-diisocyanato-4-isocyanatomethyloctane, bis(isocyanatoethyl)carbonate, bis(isocyanatoethyl)ether, lysine diisocyanatomethyl ester, lysine triisocyanate, bis(isocyanatomethyl)sulfide, bis(isocyanatoethyl)sulfide, bis(isocyanatopropyl)sulfide, bis(isocyanatohexyl)sulfide, bis(isocyanatomethyl)sulfone, bis(isocyanatomethyl)disulfide, bis(isocyanatoethyl)disulfide, bis(isocyanatopropyl)disulfide, bis(isocyanatomethylthio)methane, bis(isocyanatoethylthio)methane, bis(isocyanatomethylthio)ethane, bis(isocyanatoethylthio)ethane, 1,5-diisocyanato-2-isocyanatomethyl-3-pentane, 1,2,3-tris(isocyanatomethylthio)propane, 1,2,3-tris(isocyanatoethylthio)propane, 3,5-dithia-1,2,6,7-heptanetetraisocyanate, 2,6-diisocyanatomethyl-3,5-dithia-1,7-heptane diisocyanate, 2,5-diisocyanatomethylthiophene, 4-isocyanatoethylthio-2,6-dithia-1,8-octanediisocyanate, 1,2-diisothiocyanatoethane, and 1,6-diisothiocyanatohexane.

These may be used alone or two or more thereof may be used.

The isocyanate component preferably contains at least one (hereinafter referred to as a "preferred isocyanate compound") selected from the group consisting of bis(isocyanatomethyl)bicyclo[2.2.1]heptane, bis(isocyanatomethyl)cyclohexane, bis(isocyanatomethyl)benzene, tolylene diisocyanate, diphenylmethane diisocyanate, dicyclohexylmethane diisocyanate, hexamethylene diisocyanate, and pentamethylene diisocyanate.

Bis(isocyanatomethyl)bicyclo[2.2.1]heptane includes, for example, one or more selected from the group consisting of 2,5-bis(isocyanatomethyl)bicyclo[2.2.1]heptane and 2,6-bis(isocyanatomethyl)bicyclo[2.2.1]heptane, and is preferably a mixture of 2,5-bis(isocyanatomethyl)bicyclo[2.2.1]heptane and 2,6-bis(isocyanatomethyl)bicyclo[2.2.1]heptane.

Examples of bis(isocyanatomethyl)cyclohexane include 1,3-bis(isocyanatomethyl)cyclohexane and 1,4-bis(isocyanatomethyl)cyclohexane. Among these, 1,3-bis(isocyanatomethyl)cyclohexane is preferable.

Examples of bis(isocyanatomethyl)benzene include 1,3-bis(isocyanatomethyl)benzene and 1,4-bis(isocyanatomethyl)benzene. Among these, 1,3-bis(isocyanatomethyl)benzene is preferable.

Examples of tolylene diisocyanates include 2,4-tolylene diisocyanate and 2,6-tolylene diisocyanate. Among these, 2,4-tolylene diisocyanate is preferable.

Examples of diphenylmethane diisocyanates include 4,4'-diphenylmethane diisocyanate and 2,4'-diphenylmethane diisocyanate.

Examples of dicyclohexylmethane diisocyanates include dicyclohexylmethane-4,4'-diisocyanate.

In the isocyanate component, the content of the above "preferred isocyanate compound" is preferably 80 mass% or more, more preferably 90 mass% or more, and still more preferably 95 mass% or more and 100 mass% or less.

### (Active Hydrogen Compound Component)

Examples of active hydrogen compound components include polythiol components, polyol components, and polyamine components.

### (Polythiol Component)

Examples of polythiol components include ester compounds of a polyol compound and a carboxylic acid compound containing mercapto groups, linear or branched aliphatic polythiol compounds, polythiol compounds having an aliphatic ring, and polythiol compounds having an aromatic ring.

In the ester compound of a polyol compound and a carboxylic acid compound containing mercapto groups, examples of polyol compounds include compounds containing two or more hydroxyl groups in the molecule. Here, examples of polyol compounds include ethylene glycol, diethylene glycol, propanediol, propanetriol, butanediol, trimethylolpropane, bis(2-hydroxyethyl)disulfide, pentaerythritol, and dipentaerythritol.

Examples of carboxylic acid compounds containing mercapto groups include thioglycolic acid, mercaptopropionic acid, thiolactic acid compounds, and thiosalicylic acid.

Examples of ester compounds of a polyol compound and a carboxylic acid compound containing mercapto groups include ethylene glycol bis(2-mercaptoacetate), ethylene glycol bis(3-mercaptopropionate), diethylene glycol bis(2-mercaptoacetate), diethylene glycol bis(3-mercaptopropionate), 1,4-butanediol bis(2-mercaptoacetate), 1,4-butanediol bis(3-mercaptopropionate), trimethylolpropane tris(2-mercaptoacetate), trimethylolpropane tris(3-mercaptopropionate), pentaerythritol tetrakis(2-mercaptoacetate), pentaerythritol tetrakis(3-mercaptopropionate), dipentaerythritol hexakis(2-mercaptoacetate), and dipentaerythritol hexakis(3-mercaptopropionate).

Examples of linear or branched aliphatic polythiol compounds include 1,2-ethanedithiol, 1,1-propanedithiol, 1,2-propanedithiol, 1,3-propanedithiol, 2,2-propanedithiol, 1,6-hexanedithiol, 1,2,3-propanetrithiol, 2,2-dimethylpropane-1,3-dithiol, 3,4-dimethyloxybutane-1,2-dithiol, 2,3-dimercapto-1-propanol, 1,2-dimercaptopropyl methyl ether, 2,3-dimercaptopropyl methyl ether, dimercaptoethyl ether, 2-(2-mercaptoethylthio)propane-1,3-dithiol, 2,2-bis(mercaptomethyl)-1,3-propanedithiol, bis(mercaptomethylthio)methane, tris(mercaptomethylthio)methane, bis(2-mercaptoethylthio)methane, 1,2-bis(mercaptomethylthio)ethane, 1,2-bis(2-mercaptoethylthio)ethane, 1,3-bis(mercaptomethylthio)propane, 1,3-bis(2-mercaptoethylthio)propane, 1,1,2,2-tetrakis(mercaptoethylthio)ethane, 1,1,3,3-tetrakis(mercaptoethylthio)propane, 3-mercaptomethyl-1,5-dimercapto-2,4-dithiapentane, tetrakis(mercaptoethylthio)propane, bis(2-mercaptoethyl)ether, bis(2-mercaptoethyl)sulfide, bis(2-mercaptoethyl)disulfide, 1,2-bis(2-mercaptoethylthio)-3-mercaptopropane, 4,7-bis(mercaptomethyl)-3,6,9-trithiaundecane-1,11-dithiol, 4,8-bis(mercaptomethyl)-3,6,9-trithiaundecane-1,11-dithiol, and 5,7-bis(mercaptomethyl)-3,6,9-trithiaundecane-1,11-dithiol.

Examples of polythiol compounds having an aliphatic ring include 1,1-cyclohexanedithiol, 1,2-cyclohexanedithiol, methylcyclohexanedithiol, bis(mercaptomethyl)cyclohexane, 2-(2,2-bis(mercaptomethylthio)ethyl)-1,3-dithiethane, 2,5-bis(mercaptomethyl)-1,4-dithiane, and 4,8-bis(mercaptomethyl)-1,3-dithiane.

Examples of polythiol compounds having an aromatic ring include 1,3-dimercaptobenzene, 1,4-dimercaptobenzene, 1,3-bis(mercaptomethyl)benzene, 1,4-bis(mercaptomethyl)benzene, 1,3-bis(mercaptoethyl)benzene, 1,4-bis(mercaptoethyl)benzene, 1,3,5-trimercaptobenzene, 1,3,5-tris(mercaptomethyl)benzene, 1,3,5-tris(mercaptoethyl)benzene, 4,4'-dimercaptobiphenyl, 4,4'-dimercaptobibenzyl, 2,5-toluenedithiol, 1,5-naphthalenedithiol, 2,6- naphthalenedithiol, 2,7-naphthalenedithiol, 2,4-dimethylbenzene-1,3-dithiol, 4,5-dimethylbenzene-1,3-dithiol, 9,10-anthracenedimethanethiol, 1,3-di(p-methyloxyphenyl)propane-2,2-dithiol, 1,3-diphenylpropane-2,2-dithiol, phenylmethane-1,1-dithiol, and 2,4-di(p-mercaptophenyl)pentane.

These may be used alone or two or more thereof may be used.

### (Polyol Component)

Examples of polyol components include ethylene glycol, diethylene glycol, propanediol, propanetriol, butanediol, trimethylolpropane, bis(2-hydroxyethyl)disulfide, pentaerythritol, and dipentaerythritol.

### (Polyamine Component)

Examples of polyamine components include polymethylenediamine, polyetherdiamine, diethylenetriamine, iminobispropylamine, bishexamethylenetriamine, diethylenetriamine, tetraethylenepentamine, pentaethylenehexaamine, pentaethylenehexaamine, dimethylaminopropylamine, aminoethylethanolamine, methyliminobispropylamine, methanediamine, N-aminomethylbiperazine, 1,3-diaminocyclohexane, isophoronediamine, meta-xylenediamine, tetrachloroparaxylylenediamine, metaphenylenediamine, 4,4'-methylenedianiline, diaminodiphenylsulfone, benzidine, diaminodiphenyl ether, 4,4'-thiodianiline, 4,4'-bis(o-toluidine)dianisidine, o-phenylenediamine, 2,4-toluenediamine, 2,5-toluenediamine, methylenebis(o-chloroaniline), diaminiditolylsulfone, bis(3,4-diaminophenyl)sulfone, 2,6-diaminopyridine, 4-chloro-o-phenylenediamine, 4-methoxy-6-methyl-m-phenylenediamine, m-aminobenzylamine, N,N,N',N'-tetramethyl-1,3-butanediamine, N,N,N',N'-tetramethyl-p-phenylenediamine, tetramethylguanidine, 2-dimethylamino-2-hydroxypropane, pyrazine, 2,4,6-tris(dimethylaminomethylol)phenol, N-methylpiperazine, N-β(aminoethyl)γ-aminopropyltrimethoxysilane, N-β(aminoethyl)γ-aminopropylmethyldimethoxysilane, and γ-aminopropylmethyldimethoxysilane.

The active hydrogen compound component includes preferably at least one selected from the group consisting of toluenediamine, pentaerythritol tetrakismercaptoacetate, pentaerythritol tetrakismercaptopropionate, trimethylolpropane trismercaptoacetate, trimethylolpropane trismercaptopropionate, bis(mercaptoethylthio)mercaptopropane, bis(mercaptomethyl)-3,6,9-trithiaundecandithiol, dimercaptoethyl sulfide, bis(mercaptomethyl)dithiane, dimercaptoethyl ether and diethylene glycol.

Examples of toluenediamines include 2,4-toluenediamine and 2,5-toluenediamine.

Examples of pentaerythritol tetrakis(mercaptoacetate) include pentaerythritol tetrakis(2-mercaptoacetate).

Examples of pentaerythritol tetrakis(mercaptopropionate) include pentaerythritol tetrakis(3-mercaptopropionate).

Examples of trimethylolpropane tris(mercaptoacetate) include trimethylolpropane tris(2-mercaptoacetate).

Examples of trimethylolpropane tris(mercaptopropionate) include trimethylolpropane tris(3-mercaptopropionate).

Examples of bis(mercaptoethylthio)mercaptopropane include 1,2-bis(2-mercaptoethylthio)-3-mercaptopropane.

Examples of bis(mercaptomethyl)-3,6,9-trithiaundecanedithiol include 4,7-bis(mercaptomethyl)-3,6,9-trithiaundecane-1,11-dithiol, 4,8-bis(mercaptomethyl)-3,6,9-trithiaundecane-1,11-dithiol, 5,7-bis(mercaptomethyl)-3,6,9-trithiaundecane-1,11-dithiol. Bis(mercaptomethyl)-3,6,9-trithiaundecanedithiol is preferably a mixture of 4,7-bis(mercaptomethyl)-3,6,9-trithiaundecane-1,11-dithiol, 4,8-bis(mercaptomethyl)-3,6,9-trithiaundecane-1,11-dithiol, and 5,7-bis(mercaptomethyl)-3,6,9-trithiaundecane-1,11-dithiol.

The active hydrogen compound component is preferably a polythiol component.

The polythiol component
includes preferably at least one selected from the group consisting of 2,5-bis(mercaptomethyl)-1,4-dithiane, pentaerythritol tetrakis(2-mercaptoacetate), pentaerythritol tetrakis(3-mercaptopropionate), 4-mercaptomethyl-1,8-dimercapto-3,6-dithiaoctane, 4,7-bis(mercaptomethyl)-3,6,9-trithiaundecane-1,11-dithiol, 4,8-bis(mercaptomethyl)-3,6,9-trithiaundecane-1,11-dithiol, 5,7-bis(mercaptomethyl)-3,6,9-trithiaundecane-1,11-dithiol, trimethylolpropane tris(2-mercaptoacetate), trimethylolpropane tris(3-mercaptopropionate), butanediol bis(2-mercaptoacetate), butanediol bis(3-mercaptopropionate), dipentaerythritol hexakis(2-mercaptoacetate), and dipentaerythritol hexakis(3-mercaptopropionate);
more preferably at least one selected from the group consisting of 4,7-bis(mercaptomethyl)-3,6,9-trithiaundecane-1,11-dithiol, 4,8-bis(mercaptomethyl)-3,6,9-trithiaundecane-1,11-dithiol, 5,7-bis(mercaptomethyl)-3,6,9-trithiaundecane-1,11-dithiol, 4-mercaptomethyl-1,8-dimercapto-3,6-dithiaoctane, pentaerythritol tetrakis(3-mercaptopropionate), 2,5-bis(mercaptomethyl)-1,4-dithiane, and pentaerythritol tetrakis(2-mercaptoacetate) ;
still more preferably at least one selected from the group consisting of 4,7-bis(mercaptomethyl)-3,6,9-trithiaundecane-1,11-dithiol, 4,8-bis(mercaptomethyl)-3,6,9-trithiaundecane-1,11-dithiol, and 5,7-bis(mercaptomethyl)-3,6,9-trithiaundecane-1,11-dithiol; and
yet more preferably a mixture of 4,7-bis(mercaptomethyl)-3,6,9-trithiaundecane-1,11-dithiol, 4,8-bis(mercaptomethyl)-3,6,9-trithiaundecane-1,11-dithiol, and 5,7-bis(mercaptomethyl)-3,6,9-trithiaundecane-1,11-dithiol.

The amount of the above preferable polythiol component in the polythiol component is preferably 50 mass% or more, more preferably 70 mass% or more, still more preferably 90 mass% or more, and yet more preferably 95 mass% or more, and 100 mass% or less.

The equivalent ratio of mercapto groups of the polythiol component and isocyanato groups of the polyisocyanate component (mercapto group/isocyanato group) is preferably 40/60 or more, more preferably 43/57 or more, still more preferably 45/55 or more, and preferably 60/40 or less, more preferably 55/45 or less, and still more preferably 53/47 or less.

A total content of the polythiol component and the polyisocyanate component in the polymerizable composition is preferably 80 mass% or more, more preferably 90 mass% or more, still more preferably 95 mass% or more, and 100 mass% or less.

### (Episulfide Resin)

The episulfide resin is a cured product of a polymerizable composition containing an epithio compound. Here, the polymerizable composition may contain other monomers.

### (Epithio Compound)

The epithio compound is a compound having episulfide groups (epithio groups).

Examples of epithio compounds include episulfide compounds having a linear or branched aliphatic framework, episulfide compounds having an alicyclic framework, episulfide compounds having an aromatic framework, and episulfide compounds having a dithiane ring framework.

Examples of episulfide compounds having a linear or branched aliphatic framework include bis-(β-epithiopropyl)sulfide, bis-(β-epithiopropyl)disulfide, 2-(2-β-epithiopropylthioethylthio)-1,3-bis(β-epithiopropylthio)propane, 1,2-bis[(2-β-epithiopropylthioethyl)thio]-3-(β-epithiopropylthio)propane, tetrakis(β-epithiopropylthiomethyl)methane, and 1,1,1-tris(β-epithiopropylthiomethyl)propane.

Examples of episulfide compounds having an alicyclic framework include 1,3-bis(β-epithiopropylthio)cyclohexane, 1,4-bis(β-epithiopropylthio)cyclohexane, 1,3-bis(β-epithiopropylthiomethyl)cyclohexane, 1,4-bis(β-epithiopropylthiomethyl)cyclohexane, bis[4-(β-epithiopropylthio)cyclohexyl]methane, 2,2-bis[4-(β-epithiopropylthio)cyclohexyl]propane, and bis[4-(β-epithiopropylthio)cyclohexyl]sulfide.

Examples of episulfide compounds having an aromatic framework include 1,3-bis(β-epithiopropylthio)benzene, 1,4-bis(β-epithiopropylthio)benzene, 1,3-bis(β-epithiopropylthiomethyl)benzene, 1,4-bis(β-epithiopropylthiomethyl)benzene, bis[4-(β-epithiopropylthio)phenyl]methane, 2,2-bis[4-(β-epithiopropylthio)phenyl]propane, bis[4-(β-epithiopropylthio)phenyl]sulfide, bis[4-(β-epithiopropylthio)phenyl]sulfine, and 4,4-bis(β-epithiopropylthio)biphenyl.

Examples of episulfide compounds having a dithiane ring framework include 2,5-bis(β-epithiopropylthiomethyl)-1,4-dithiane, 2,5-bis(β-epithiopropylthioethylthiomethyl)-1,4-dithiane, 2,5-bis(β-epithiopropylthioethyl)-1,4-dithiane, and 2,3,5-tri(β-epithiopropylthioethyl)-1,4-dithiane.

In addition to the epithio compound, other polymerizable components such as the above polyisocyanate component and polythiol component may be added.

Among these, an episulfide compound having a linear or branched aliphatic framework is preferable, and bis(β-epithiopropyl) sulfide or bis(β-epithiopropyl)disulfide is more preferable.

The content of the epithio compound in the polymerizable composition is preferably 50 mass% or more, more preferably 60 mass% or more, still more preferably 70 mass% or more, yet more preferably 80 mass% or more, and even more preferably 90 mass% or more, and preferably 98 mass% or less and more preferably 96 mass% or less.

The polymerizable composition preferably further contains sulfur or a polythiol compound in combination with an epithio compound.

The content of sulfur in the polymerizable composition is preferably 1 mass% or more, more preferably 5 mass% or more, still more preferably 10 mass% or more, and preferably 30 mass% or less, and more preferably 20 mass% or less.

Examples of polythiol compounds include the compounds exemplified above.

When used in combination with an epithio compound, the content of the polythiol compound in the polymerizable component is preferably 2 mass% or more, more preferably 4 mass% or more, and preferably 50 mass% or less, more preferably 40 mass% or less, still more preferably 30 mass% or less, yet more preferably 20 mass% or less, and even more preferably 10 mass% or less.

When the polymerizable composition contains a polyisocyanate component and a polythiol component or an epithio compound, it preferably contains a polymerization catalyst.

Examples of polymerization catalysts include tin compounds and nitrogen-containing compounds.

Examples of tin compounds include alkyltin compounds and alkyltin halide compounds.

Examples of alkyltin compounds include dibutyltin diacetate and dibutyltin dilaurate.

Examples of alkyltin halide compounds include dibutyltin dichloride, dimethyltin dichloride, monomethyltin trichloride, trimethyltin chloride, tributyltin chloride, tributyltin fluoride, and dimethyltin dibromide.

Among these, dibutyltin diacetate, dibutyltin dilaurate, dibutyltin dichloride, and dimethyltin dichloride are preferable, and dimethyltin dichloride is more preferable.

Examples of nitrogen-containing compounds include tertiary amines, quaternary ammonium salts, imidazole compounds, and pyrazole compounds. Tertiary amines are preferably hindered amines.

Examples of tertiary amines include triethylamine, tri-n-propylamine, triisopropylamine, tri-n-butylamine, triisobutylamine, N,N-dimethylbenzylamine, N-methylmorpholine, N,N-dimethylcyclohexylamine, pentamethyldiethylenetriamine, bis(2-dimethylaminoethyl) ether, N-methylmorpholine, N,N'-dimethylpiperazine, N,N,N',N'-tetramethylethylenediamine, and 1,4-diazabicyclo[2.2.2]octane(DABCO).

Examples of hindered amines include 1,2,2,6,6-pentamethyl-4-piperidinol, 1,2,2,6,6-pentamethyl-4-hydroxyethyl-4-piperidinol, methyl-1,2,2,6,6-pentamethyl-4-piperidyl sebacate, a mixture of methyl-1,2,2,6,6-pentamethyl-4-piperidyl sebacate and bis(1,2,2,6,6-pentamethyl-4-piperidyl)sebacate, bis(1,2,2,6,6-pentamethyl-4-piperidyl)sebacate, bis(2,2,6,6-tetramethyl-1-(octyloxy)-4-piperidyl)sebacate, bis(1,2,2,6,6-pentamethyl-4-piperidyl) [[3,5-bis(1,1-dimethylethyl)-4-hydroxyphenyl]methyl]butylmalonate, and tetrakis(1,2,2,6,6-pentamethyl-4-piperidyl)butane-1,2,3,4-tetracarboxylate.

Examples of quaternary ammonium salts include tetraethylammonium hydroxide.

Examples of imidazole compounds include imidazole, 1-methyl-2-mercapto-1H-imidazole, 1,2-dimethylimidazole, benzylmethylimidazole, and 2-ethyl-4-imidazole.

Examples of pyrazole compounds include pyrazole and 3,5-dimethylpyrazole.

Among these, tertiary amines such as hindered amines, imidazole compounds, and pyrazole compounds are preferable, imidazole compounds are more preferable, and 1-methyl-2-mercapto-1H-imidazole is still more preferable.

When an isocyanate component and an active hydrogen compound component are contained, the amount of the polymerization catalyst added in the polymerizable composition with respect to a total amount of 100 parts by mass of the isocyanate component and the active hydrogen compound component is preferably 0.001 parts by mass or more, more preferably 0.005 parts by mass or more, still more preferably 0.007 parts by mass or more, and preferably 2 parts by mass or less, more preferably 1 part by mass or less, and still more preferably 0.5 parts by mass or less.

When an epithio compound is contained, the amount of the polymerization catalyst added in the polymerizable composition with respect to a total amount of 100 parts by mass of the polymerizable component is preferably 0.001 parts by mass or more, more preferably 0.005 parts by mass or more, still more preferably 0.007 parts by mass or more, and preferably 2 parts by mass or less, more preferably 1 part by mass or less, and still more preferably 0.5 parts by mass or less.

### (Polycarbonate Resin)

The polycarbonate resin is preferably a cured product of a polymerizable composition containing diethylene glycol bisallyl carbonate.

In order to obtain a three-dimensionally crosslinked optical resin, the monomers preferably include a monomer having two or more polymerizable unsaturated bonds in the molecule.

Examples of polymerizable unsaturated bonds include (meth)acrylate groups, allyl groups, and vinyl groups. Here, the (meth)acrylate group is at least one selected from the group consisting of methacrylate groups and acrylate groups.

Among these, at least one selected from the group consisting of methacrylate groups and allyl groups is preferable.

Monomers having two or more polymerizable unsaturated bonds in the molecule include preferably diethylene glycol bisallyl carbonate and more preferably diethylene glycol bisallyl carbonate, benzyl methacrylate, diallyl phthalate and an alkyl methacrylate having 1 to 4 carbon atoms in the alkyl group.

The amount of diethylene glycol bisallyl carbonate added with respect to a total amount of monomers is preferably 5 mass% or more, more preferably 10 mass% or more, still more preferably 20 mass% or more, and preferably 100 mass% or less, more preferably 80 mass% or less, still more preferably 50 mass% or less, and yet more preferably 40 mass% or less.

When used in combination with benzyl methacrylate, diallyl phthalate and an alkyl methacrylate having 1 to 4 carbon atoms in the alkyl group, the amount of diethylene glycol bisallyl carbonate added with respect to a total amount of monomers is more preferably 5 mass% or more, more preferably 10 mass% or more, still more preferably 20 mass% or more, and more preferably 40 mass% or less, and more preferably 35 mass% or less.

The amount of benzyl methacrylate added with respect to a total amount of monomers is preferably 5 mass% or more, more preferably 10 mass% or more, still more preferably 15 mass% or more, and preferably 40 mass% or less, more preferably 30 mass% or less, and still more preferably 25 mass% or less.

As the diallyl phthalate, one or two selected from the group consisting of diallyl isophthalate and diallyl terephthalate may be exemplified.

The amount of diallyl phthalate added with respect to a total amount of monomers is preferably 14 mass% or more, more preferably 20 mass% or more, still more preferably 30 mass% or more, and preferably 88 mass% or less, more preferably 70 mass% or less, and still more preferably 60 mass% or less.

As the alkyl methacrylate having 1 to 4 carbon atoms in the alkyl group, at least one selected from the group consisting of methyl methacrylate, ethyl methacrylate, n-propyl methacrylate, iso-propyl methacrylate, n-butyl methacrylate, sec-butyl methacrylate, iso-butyl methacrylate, and tert-butyl methacrylate may be exemplified.

The amount of alkyl methacrylate added with respect to a total amount of monomers is preferably 1 mass% or more, more preferably 2 mass% or more, still more preferably 3 mass% or more, and preferably 6 mass% or less, and more preferably 5 mass% or less.

Examples of radical initiators used for polymerization include 1,1-azobiscyclohexane carbonate, diisopropyl peroxycarbonate, 1,1'-azobiscyclohexane nitrate, and di-tert-butyl peroxide.

The amount of the radical initiator added with respect to 100 parts by mass of monomers is preferably 0.1 parts by mass or more, more preferably 0.5 parts by mass or more, and still more preferably 1.0 part by mass or more, and preferably 10 parts by mass or less, more preferably 8 parts by mass or less, and still more preferably 5 parts by mass or less.

### (Acrylic Resin)

The acrylic resin is a cured product of a polymerizable composition containing an acrylic compound. Here, the polymerizable composition may contain other monomers.

Examples of acrylic compounds include polyfunctional (meth)acrylate compounds having an aromatic ring, a polyalkylene glycol di(meth)acrylate, and monofunctional acrylates.

Among these, it is preferable to include a polyfunctional (meth)acrylate compound having an aromatic ring and a polyalkylene glycol di(meth)acrylate.

Examples of polyfunctional (meth)acrylate compounds having an aromatic ring include an alkylene oxide-modified bisphenol A having a (meth)acryloyl group at both ends and an alkylene oxide-modified and urethane-modified bisphenol A having a (meth)acryloyl group at both ends.

Among these, an alkylene oxide-modified bisphenol A having a (meth)acryloyl group at both ends is preferable.

The alkylene oxide-modified bisphenol A having a (meth)acryloyl group at both ends is preferably a compound represented by Formula (51): 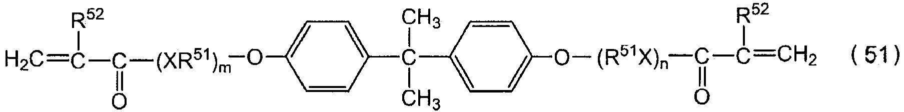 [where, R⁵¹ is an ethylene group or a propylene group, R⁵² is a hydrogen atom or a methyl group, X is an oxygen atom or a sulfur atom, and preferably an oxygen atom, m and n are an average number of moles added, and m+n is 1.5 to 6, and preferably 2 to 4].

Examples of alkylene oxide-modified bisphenol A having a (meth)acryloyl group at both ends include 2,2-bis[4-[2-((meth)acryloyloxy)ethoxy]phenyl]propane and 2,2-bis[4-[2-((meth)acryloyloxy)ethoxy]-3,5-dibromophenyl]propane.

The content of the polyfunctional (meth)acrylate compound having an aromatic ring in the polymerizable composition is preferably 40 mass% or more, more preferably 50 mass% or more, still more preferably 55 mass% or more, and preferably 90 mass% or less, more preferably 80 mass% or less, and still more preferably 70 mass% or less.

Examples of polyalkylene glycol di(meth)acrylate include diethylene glycol di(meth)acrylate, triethylene glycol di(meth)acrylate, tetraethylene glycol di(meth)acrylate, dipropylene glycol di(meth)acrylate, tripropylene glycol di(meth)acrylate, tetrapropylene glycol di(meth)acrylate, dibutylene glycol di(meth)acrylate, tributylene glycol di(meth)acrylate, and tetrabutylene glycol di(meth)acrylate.

The content of polyalkylene glycol di(meth)acrylate in the polymerizable composition is preferably 10 mass% or more, more preferably 20 mass% or more, still more preferably 30 mass% or more, and preferably 60 mass% or less, more preferably 50 mass% or less, and still more preferably 45 mass% or less.

Examples of monofunctional (meth)acrylates include phenyl(meth)acrylate, benzyl(meth)acrylate, phenoxyethyl(meth)acrylate, 3-phenoxy-2-hydroxypropyl(meth)acrylate, 2-phenylphenyl(meth)acrylate, 4-phenylphenyl(meth)acrylate, 3-(2-phenylphenyl)-2-hydroxypropyl(meth)acrylate, 3-(4-phenylphenyl)-2-hydroxypropyl(meth)acrylate, 1-naphthyloxyethyl(meth)acrylate, 2-naphthyloxyethyl(meth)acrylate, 2,4,6-tribromophenyl(meth)acrylate, 2,4,6-tribromophenoxyethyl(meth)acrylate, 2,4,6-tribromophenyl-di(oxyethyl)-(meth)acrylate, and 2,4,6-tribromobenzyl(meth)acrylate.

A total content of the polymerizable component in the polymerizable composition is preferably 80 mass% or more, more preferably 85 mass% or more, still more preferably 90 mass% or more, and preferably 99 mass% or less, and more preferably 95 mass% or less.

When the polymerizable composition contains an acrylic compound, it preferably contains a radical polymerization initiator.

Examples of radical polymerization initiators include energy ray-sensitive polymerization initiators and heat-sensitive polymerization initiators.

Examples of energy ray-sensitive polymerization initiators include 2-hydroxy-2-methyl-1-phenylpropan-1-one, hydroxycyclohexyl phenyl ketone, methyl phenylglyoxylate, and 2,4,6-trimethylbenzoyldiphenylphosphine oxide.

Examples of heat-sensitive polymerization initiators include organic peroxides and azo compounds.

Examples of organic peroxide include peroxy esters such as tert-butyl peroxyneodecanoate, tert-butyl peroxypivalate, tert-butyl peroxyisobutyrate, tert-butyl peroxyacetate, cumyl peroxyneodecanoate, tert-butyl peroxyoxtoate, tert-butyl peroxyisopropylcarbonate, cumyl peroxyoxtoate, tert-hexyl peroxyneodecanoate, tert-hexyl peroxypivalate, and tert-butyl peroxyneohexanoate; peroxy ketals such as 1,1-bis(tert-butyl peroxy)-3,3,5-trimethylcyclohexane, 1,1-bis(tert-butylperoxy)cyclohexane, 2,2-bis(tert-butylperoxy)octane, and 2,2-bis(tert-butylperoxy)butane; diacyl peroxides such as acetyl peroxide, isobutyryl peroxide, octanoyl peroxide, lauroyl peroxide, benzoyl peroxide, and m-toluoyl peroxide; and peroxydicarbonates such as diisopropyl peroxydicarbonate and di-n-propyl peroxydicarbonate.

Examples of azo compounds include 2,2'-azobisisobutyrolnitrile, 1,1'-azobis(cyclohexane-1-carbonitrile), 2,2'-azobis(2-methylbutyronitrile), 2,2'-azobis(2,4-dimethylvaleronitrile), dimethyl-2,2'-azobisisobutyrate, and 2,2'-azobis(2,4,4-trimethylpentane).

The amount of the radical polymerization initiator added with respect to a total amount of 100 parts by mass of the acrylic compound is preferably 0.01 parts by mass or more, more preferably 0.1 parts by mass or more, still more preferably 0.5 parts by mass or more, and preferably 10 parts by mass or less, more preferably 5 parts by mass or less, and still more preferably 3 parts by mass or less.

The lens substrate may contain other additives such as a mold releasing agent, a coloring agent, an antioxidant, an anti-coloring agent, and a fluorescent brightening agent. These may be used alone or two or more thereof may be used.

### [Mold Releasing Agent]

Examples of mold releasing agents include phosphate ester compounds such as isopropyl acid phosphate, butyl acid phosphate, octyl acid phosphate, nonyl acid phosphate, decyl acid phosphate, isodecyl acid phosphate, isodecyl acid phosphate, tridecyl acid phosphate, stearyl acid phosphate, propyl phenyl acid phosphate, butyl phenyl acid phosphate, and butoxyethyl acid phosphate. The phosphate ester compound may be either a phosphate mono-ester compound or a phosphate di-ester compound, and is preferably a mixture of a phosphate mono-ester compound and a phosphate di-ester compound.

The amount of the mold releasing agent added with respect to a total amount of 100 parts by mass of the resin is preferably 0.01 parts by mass or more, preferably 0.05 parts by mass or more, and preferably 1.00 part by mass or less, and preferably 0.50 parts by mass or less.

### [Coloring Agent]

The lens substrate may contain a coloring agent in a range in which the luminous transmittance to be described below is not impaired.

In order to make coloration caused by addition of the above Compound 1 inconspicuous, the lens substrate may contain a coloring agent L having a maximum absorption wavelength of 550 nm or more and 600 nm or less (hereinafter simply referred to as a "coloring agent L") at 20 ppm by mass in a toluene solution.

In order to make coloration caused by addition of the above Compound 1 inconspicuous, the lens substrate may contain a coloring agent S having a maximum absorption wavelength of 500 nm or more and less than 550 nm (hereinafter simply referred to as a "coloring agent S") at 20 ppm by mass in a toluene solution.

### (Coloring Agent L)

In order to obtain a slightly bluish lens substrate with a favorable color tone, the coloring agent L has a maximum absorption wavelength of 550 nm or more and 600 nm or less at 20 ppm by mass in a toluene solution. Here, 20 ppm by mass in a toluene solution means the proportion of the solute with respect to the entire toluene solution.

In addition, in order to obtain a slightly bluish lens substrate with a favorable color tone, the maximum absorption wavelength of the coloring agent L is preferably 550 nm or more, preferably 560 nm or more, and preferably 580 nm or more. Here, in order to obtain a slightly bluish resin composition with a favorable color tone, the maximum absorption wavelength of the coloring agent L is preferably 600 nm or less, and preferably 590 nm or less.

Examples of coloring agents L include C.I. Solvent Violet 11, 13, 14, 26, 31, 33, 36, 37, 38, 45, 47, 48, 51, 59, and 60; and C.I. Disperse Violet 26, 27, 28. Among these, C.I. Disperse Violet 27 and C.I. Solvent Violet 13, 31 are preferable, and in consideration of high stability and little change in the color tone even when the polymerizable composition is polymerized, C.I. Disperse Violet 27 and C.I. Solvent Violet 13 are more preferable, and C.I. Disperse Violet 27 is still more preferable.

In order to obtain a slightly bluish lens substrate with a favorable color tone, the amount of the coloring agent L added with respect to the resin is preferably 10,000 ppb by mass or less, more preferably 3,000 ppb by mass or less, and still more preferably 1,500 ppb by mass or less. In order to obtain a slightly bluish lens substrate with a favorable color tone, the amount of the coloring agent L added is preferably 200 ppb by mass or more, more preferably 300 ppb by mass or more, and still more preferably 400 ppb by mass or more.

### (Coloring Agent S)

In order to obtain a slightly bluish lens substrate with a favorable color tone, the coloring agent S has a maximum absorption wavelength of 500 nm or more and less than 550 nm at 20 ppm by mass in a toluene solution.

In addition, in order to obtain a slightly bluish lens substrate with a favorable color tone, the maximum absorption wavelength of the coloring agent S is preferably 500 nm or more, more preferably 510 nm or more, and still more preferably 530 nm or more. Here, in order to obtain a slightly bluish lens substrate with a favorable color tone, the maximum absorption wavelength of the coloring agent L is preferably 545 nm or less.

In order to obtain a slightly bluish lens substrate with a favorable color tone, examples of coloring agents S include C.I. Solvent Red 24, 49, 52, 90, 91, 111, 118, 119, 122, 124, 125, 127, 130, 132, 143, 145, 146, 150, 151, 155, 160, 168, 169, 172, 175, 181, 207, 218, 222, 227, 230, 245, 247; and C.I. Acid Red 73, 80, 91, 92, 97, 138, 151, 211, 274, 289. Among these, C.I. Solvent Red 52 and 146 are preferable, and in consideration of high stability and little change in the color tone even when the polymerizable composition is polymerized, C.I. Solvent Red 52 is more preferable.

In order to obtain a slightly bluish lens substrate with a favorable color tone, the amount of the coloring agent S added with respect to the resin is preferably 500 ppb by mass or less, more preferably 100 ppb by mass or less, and still more preferably 50 ppb by mass or less. In order to obtain a slightly bluish lens substrate with a favorable color tone, the amount of the coloring agent S added is preferably 1 ppb by mass or more, more preferably 3 ppb by mass or more, and still more preferably 5 ppb by mass or more.

The mass ratio of the coloring agent L and the coloring agent S [the mass of the coloring agent L/the mass of the coloring agent S] is preferably 5 or more and 500 or less in order to obtain a slightly bluish lens substrate with a favorable color tone.

The mass ratio of the coloring agent L and the coloring agent S is preferably 5 or more, more preferably 10 or more, still more preferably 15 or more, and yet more preferably 20 or more. In addition, the mass ratio of the coloring agent L and the coloring agent S is preferably 500 or less, more preferably 200 or less, still more preferably 100 or less, and yet more preferably 80 or less.

### [Structure of Lens Substrate, etc.]

The lens substrate may be either a finished lens or a semi-finished lens.

The surface shape of the lens substrate is not particularly limited, and may be flat, convex, concave or the like.

The lens substrate may be used for any application such as a single focal lens, a multifocal lens, and a progressive power lens. For example, as one example, for a progressive power lens, usually, a near portion region (near portion) and a progressive portion region (intermediate region) are included in the above lower region, and a distance portion region (distance portion) is included in an upper region.

As the lens substrate, a colorless lens substrate is usually used, but a lens substrate that is colored in a range in which the transparency is not impaired can also be used.

The lens substrate is preferably of a meniscus type. A "meniscus type" lens substrate means a lens substrate having a curved surface formed on both sides. When the meniscus type lens substrate contains the above Compound 1, it is possible to reduce astigmatism.

The optical center thickness of the lens substrate is not particularly limited, and is preferably 0.5 mm or more and 10.0 mm or less, more preferably 0.5 mm or more and 5.0 mm or less, still more preferably 0.5 mm or more and 3.0 mm or less, and yet more preferably 0.5 mm or more and 2.0 mm or less.

The diameter of the lens substrate is not particularly limited, and is usually about 50 to 100 mm.

The refractive index ne of the lens substrate is preferably 1.52 or more, more preferably 1.53 or more, still more preferably 1.55 or more, yet more preferably 1.58 or more, and even more preferably 1.60 or more.

In order to enhance an effect of improving the Abbe number according to the inclusion of Compound 1, the refractive index ne of the lens substrate is preferably 1.70 or more, and more preferably 1.74 or more.

Here, although the upper limit of the refractive index ne of the lens substrate is not particularly limited, it may be, for example 1.80 or less.

In order to reduce blue light hazard, the transmittance of light with a wavelength of 410 nm in the lens substrate is preferably 5% or less, more preferably 3% or less, and still more preferably 1.0% or less. The lower limit value of the transmittance of light with a wavelength of 410 nm is not particularly limited, and is, for example, 0.0% or more.

In order to reduce transmission of ultraviolet rays that are harmful to the eyes, the transmittance of light with a wavelength of 400 nm in the lens substrate is preferably 3% or less, more preferably 1% or less, and still more preferably 0.0% or less.

The luminous transmittance of the lens substrate is preferably 70% or more, more preferably 80% or more, still more preferably 85% or more, and yet more preferably 90% or more. The upper limit value of the luminous transmittance is not particularly limited, and is, for example, 100% or less, and may be 95% or less.

The above transmittance is the transmittance at the optical center of the lens substrate and can be measured using a spectrophotometer. As the spectrophotometer, for example, a UV-Visible/NIR Spectrophotometer "UH-4150" (product name, commercially available from Hitachi High-Tech Corporation) can be used. The above transmittance can be achieved by adjusting the content of Compound 1 according to the thickness of the lens substrate.

The YI value at the optical center of the lens substrate is preferably 10.00 or less, more preferably 9.70 or less, still more preferably 9.00 or less, and yet more preferably 8.70 or less. For example, measurement can be performed at the optical center of the lens substrate using a spectrophotometer U-3500 (commercially available from Hitachi, Ltd.), and the YI value can be determined according to JIS K 7373:2006.

### [Method of Producing Lens Substrate]

Although not particularly limited, the lens substrate is obtained by, for example, a production method including
a step of curing the above polymerizable composition and
a step of annealing a cured resin.

The polymerization is preferably a cast polymerization method. For example, the lens substrate can be obtained by injecting a polymerizable composition into a mold in which a glass or metal mold and a tape or a gasket are combined and performing polymerization.

Polymerization conditions can be appropriately set according to the polymerizable composition. The polymerization start temperature is preferably 0°C or higher, more preferably 10°C or higher, and preferably 50°C or lower, and more preferably 40°C or lower. Preferably, the temperature is raised from the polymerization start temperature, and curing by heating is then performed. For example, the maximum heating temperature is usually 110°C or higher and 130°C or lower.

After polymerization is completed, the lens substrate may be released from the mold and subjected to an annealing treatment. The temperature in the annealing treatment is preferably 100 to 150°C.

### <Hard Coat Layer>

The hard coat layer is, for example, a cured film formed of a curable composition containing an inorganic oxide and a silicon compound. The curable composition preferably further contains a polyfunctional epoxy compound.

Examples of inorganic oxides include silicon oxide, aluminum oxide, titanium oxide, zirconium oxide, tungsten oxide, zinc oxide, tin oxide, beryllium oxide, antimony oxide, and composite oxides formed of two or more of these inorganic oxides. These may be used alone or two or more thereof may be used in combination. Among these inorganic oxides, silicon oxide is preferable. Here, colloidal silica may be used as the inorganic oxide.

The content of the inorganic oxide in the solid content of the curable composition is preferably 20 mass% or more and 80 mass% or less, more preferably 25 mass% or more and 70 mass% or less, and still more preferably 25 mass% or more and 50 mass% or less.

The silicon compound is, for example, a silicon compound having a hydrolyzable group such as an alkoxy group. The silicon compound is preferably a silane coupling agent containing an organic group and a hydrolyzable group bonded to silicon atoms. The organic group bonded to silicon atoms is preferably an organic group having a functional group, for example, an epoxy group such as a glycidoxy group, a vinyl group, a methacryloxy group, an acryloxy group, a mercapto group, an amino group, and a phenyl group, and more preferably an organic group having an epoxy group. Here, the silicon compound may have an alkyl group bonded to silicon.

Examples of commercial products of the above silane coupling agents include KBM-303, KBM-402, KBM-403, KBE-402, KBE-403, KBM-1403, KBM-502, KBM-503, KBE-502, KBE-503, KBM-5103, KBM-602, KBM-603, KBM-903, KBE-903, KBE-9103, KBM-573, KBM-575, KBM-9659, KBE-585, KBM-802, KBM-803, KBE-846, and KBE-9007 (product name, commercially available from Shin-Etsu Chemical Co., Ltd.).

The content of the silicon compound in the solid content of the curable composition is preferably 20 mass% or more and 90 mass% or less, more preferably 30 mass% or more and 75 mass% or less, and still more preferably 50 mass% or more and 75 mass% or less.

The polyfunctional epoxy compound is a polyfunctional epoxy compound containing two or more epoxy groups in one molecule, and more preferably a polyfunctional epoxy compound containing two or three epoxy groups in one molecule. Examples of commercial products of polyfunctional epoxy compounds include "Denacol" series EX-201, EX-211, EX-212, EX-252, EX-313, EX-314, EX-321, EX-411, EX-421, EX-512, EX-521, EX-611, EX-612, EX-614, and EX-614B (product name, commercially available from Nagase ChemteX Corporation).

The content of the polyfunctional epoxy compound in the solid content of the curable composition is preferably 0 mass% or more and 50 mass% or less, more preferably 10 mass% or more and 40 mass% or less, and still more preferably 15 mass% or more and 30 mass% or less.

The above curable composition can be prepared by mixing optional components such as an organic solvent, a leveling agent, and a curing catalyst as necessary in addition to the components described above.

The above hard coat layer can be formed by applying a curable composition to the substrate and performing a curing treatment (thermal curing, photocuring, etc.). As a method of applying a curable composition, commonly used methods such as a dipping method, a spin coating method, and a spray method can be applied. The curing treatment is usually performed by performing heating on a curable composition containing a polyfunctional epoxy compound. For example, the heat curing treatment can be performed by disposing the lens coated with the above curable composition under an environment of an atmospheric temperature of 50 to 150°C for about 30 minutes to 3 hours.

### <Foundation Layer>

For example, the above foundation layer can be formed from an aqueous resin composition containing at least one resin particle selected from the group consisting of a polyurethane resin, an acrylic resin, and an epoxy resin.

As the above aqueous resin composition, a commercially available aqueous polyurethane can be used without change or one that is diluted with an aqueous solvent as necessary can be used. Examples of commercially available aqueous polyurethanes include "Evafanol" series (product name, commercially available from Nicca Chemical Co., Ltd.), "SuperFlex" series (product name, commercially available from DKS Co., Ltd.), "Adeka Bontighter" series (product name, commercially available from ADEKA Corporation), "Olester" series (product name, commercially available from Mitsui Chemicals Inc), "Bondic" series and "Hydran" series (product name, commercially available from Dainippon Ink and Chemicals, Inc.), "Impranil" series (product name, commercially available from Bayer AG), "Soflanate" series (product name, commercially available from Japan Soflan Co., Ltd.), "Poiz" series (product name, commercially available from Kao Corporation), "Sanprene" series (product name, commercially available from Sanyo Chemical Industries, Ltd.), "Aizerax" series (product name, commercially available from Hodogaya Chemical Co., Ltd.), and "NeoRez" series (product name, commercially available from AstraZeneca).

The foundation layer can be formed, for example, by applying the above aqueous resin composition to the surface of the substrate and drying it.

### <Functional Layer>

Examples of the above functional layers include an antireflection layer, an ultraviolet light absorbing layer, an infrared light absorbing layer, a photochromic layer, an antistatic layer, and an anti-fogging layer. These functional layers may be used alone or two or more thereof may be used in combination. For these functional layers, known techniques related to spectacle lenses can be applied. Among these, it is preferable to have an antireflection layer.

### (Antireflection Layer)

For example, the antireflection layer has low refractive index layers and high refractive index layers that are alternately arranged. The number of layers that the antireflection layer has is preferably 4 to 11 and more preferably 5 to 8.

The refractive index of the low refractive index layer is preferably 1.35 to 1.80 and more preferably 1.45 to 1.50 at a wavelength of 500 to 550 nm. The low refractive index layer is formed of an inorganic oxide and preferably formed of silicon oxide.

The refractive index of the high refractive index layer is preferably 1.90 to 2.60 and more preferably 2.00 to 2.40 at a wavelength of 500 to 550 nm. The high refractive index layer is formed of, for example, an inorganic oxide. The inorganic oxide used for the high refractive index layer is preferably at least one selected from the group consisting of zirconium oxide, tantalum oxide, yttrium oxide, titanium oxide, niobium oxide and aluminum oxide, and more preferably at least one selected from the group consisting of zirconium oxide and tantalum oxide.

For the antireflection layer, low refractive index layers and high refractive index layers can be alternately laminated by a vacuum deposition method to form an antireflection layer.

### <Water-Repellent Layer>

The water-repellent layer is formed using a water-repellent material composition to be described below. The water-repellent layer may be formed on the hard coat layer or formed on the functional layer, but is preferably formed on the antireflection layer. Moreover, the water-repellent layer is preferably positioned on the outermost surface.

### <Physical Properties of Spectacle Lens>

In order to reduce blue light hazard, the transmittance of light with a wavelength of 410 nm in the entire spectacle lens is preferably 5% or less, more preferably 3% or less, and still more preferably 1.0% or less. The lower limit value of the transmittance of light with a wavelength of 410 nm is not particularly limited, and is, for example, 0.0% or more.

In order to reduce transmission of ultraviolet rays that are harmful to the eyes, the transmittance of light with a wavelength of 400 nm in the entire spectacle lens is preferably 3% or less, more preferably 1% or less, and still more preferably 0.0% or less.

The luminous transmittance of the spectacle lens is preferably 70% or more, more preferably 80% or more, still more preferably 85% or more, and yet more preferably 90% or more. The upper limit value of the luminous transmittance is not particularly limited, and is, for example, 100% or less, and may be 95% or less.

The above transmittance is the transmittance at the optical center of the spectacle lens and can be measured using a spectrophotometer. As the spectrophotometer, for example, a "UV-Visible/NIR Spectrophotometer UH-4150" (product name, commercially available from Hitachi High-Tech Corporation) can be used. The above transmittance can be achieved by adjusting the content of Compound 1 according to the thickness of the spectacle lens.

The YI value at the optical center of the spectacle lens is preferably 10.00 or less, more preferably 9.70 or less, still more preferably 9.00 or less, and yet more preferably 8.70 or less. For example, measurement can be performed at the optical center of the spectacle lens using a spectrophotometer U-3500 (commercially available from Hitachi, Ltd.), and the YI value can be determined according to JIS K 7373:2006.

As described above, the present specification discloses the following embodiments.
<1> An ultraviolet light absorbing agent containing a compound (1) represented by Formula (1): 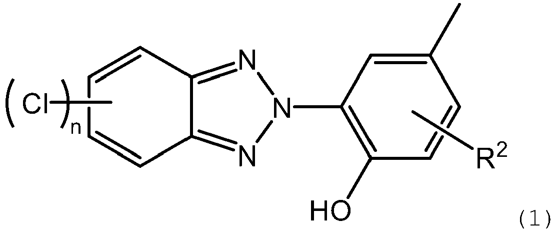 (where,
   R² is an alkyl group having 1 to 12 carbon atoms or an alkoxy group having 1 to 12 carbon atoms, and
   n is 0 or 1)
   wherein, in a spectrum of the ultraviolet light absorbing agent measured by liquid chromatography using a diode array detector, the peak area ratio of a compound (2) represented by Formula (2) is 0.008% or less: 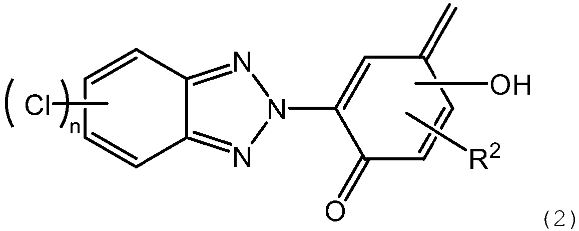 (where,
      R² is an alkyl group having 1 to 12 carbon atoms or an alkoxy group having 1 to 12 carbon atoms, and
      n is 0 or 1).
<2> The ultraviolet light absorbing agent according to <1>,
   wherein the peak area ratio of the compound (2) in a spectrum of the ultraviolet light absorbing agent measured by liquid chromatography using a diode array detector is 0.001% or more.
<3> The ultraviolet light absorbing agent according to <1> or <2>,
   wherein the peak area ratio of the compound (1) in a spectrum of the ultraviolet light absorbing agent measured by liquid chromatography using a diode array detector is 99% or more.
<4> The ultraviolet light absorbing agent according to any of <1> to <3>,
   wherein the compound (1) is a compound (1-1) represented by Formula (1-1): 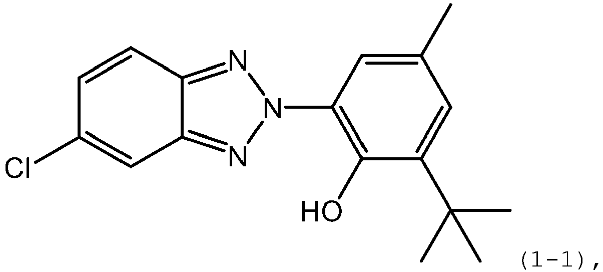 and
   the compound (2) is a compound (2-1) represented by Formula (2-1): 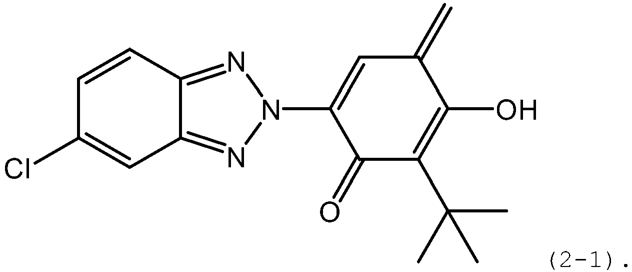
<5> A spectacle lens including a lens substrate including the ultraviolet light absorbing agent according to any of <1> to <4> and a resin.
<6> The spectacle lens according to <5>,
   wherein the resin is a cured product of an isocyanate component containing at least one selected from the group consisting of bis(isocyanatomethyl)bicyclo[2.2.1]heptane, bis(isocyanatomethyl)cyclohexane, bis(isocyanatomethyl)benzene, tolylene diisocyanate, diphenylmethane diisocyanate, dicyclohexylmethane diisocyanate, hexamethylene diisocyanate, and pentamethylene diisocyanate, and an active hydrogen compound component.
<7> The spectacle lens according to <6>,
   wherein the active hydrogen compound component contains at least one selected from the group consisting of toluenediamine, pentaerythritol tetrakismercaptoacetate, pentaerythritol tetrakismercaptopropionate, trimethylolpropane trismercaptoacetate, trimethylolpropane trismercaptopropionate, bis(mercaptoethylthio)mercaptopropane, bis(mercaptomethyl)-3,6,9-trithiaundecandithiol, dimercaptoethyl sulfide, bis(mercaptomethyl)dithiane, dimercaptoethyl ether and diethylene glycol.
<8> The spectacle lens according to <7>,
   wherein the bis(mercaptomethyl)-3,6,9-trithiaundecandithiol is a mixture of 4,7-bis(mercaptomethyl)-3,6,9-trithiaundecane-1,11-dithiol, 4,8-bis(mercaptomethyl)-3,6,9-trithiaundecane-1,11-dithiol and 5,7-bis(mercaptomethyl)-3,6,9-trithiaundecane-1,11-dithiol.
<9> The spectacle lens according to <5>,
   wherein the resin is an episulfide resin.
<10> The spectacle lens according to any of <5> to <9>, including 0.05 parts by mass or more and 2.00 parts by mass or less of the compound (1) with respect to 100 parts by mass of the resin.
<11> The spectacle lens according to any of <5> to <10>,
   wherein the lens substrate has a refractive index ne of 1.52 or more and 1.80 or less.
<12> The spectacle lens according to any of <5> to <11>,
   wherein the lens substrate has a refractive index ne of 1.70 or more and 1.80 or less.
<13> The spectacle lens according to any of <5> to <12>, including at least one layer selected from the group consisting of a hard coat layer, a foundation layer, and an antireflection layer.
<14> A method of producing ultraviolet light absorbing agent crystals containing a compound (1) represented by Formula (1): 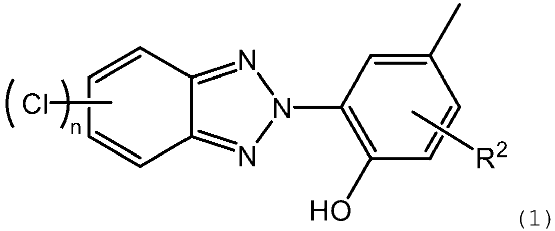 (where,
   R² is an alkyl group having 1 to 12 carbon atoms or an alkoxy group having 1 to 12 carbon atoms, and
   n is 0 or 1), the method including:
      a step of adding an isopropanol solution to a toluene solution containing the ultraviolet light absorbing agent to obtain crystals; and
      a step of washing the crystals with isopropanol.

### [Examples]

Hereinafter, the present embodiment will be described in more detail with reference to examples and comparative examples. Here, the present invention is not limited to the following examples.

### [Liquid Chromatography-Fourier Transform Mass Spectrometry (LC/MS)]

A sample was collected in a screw bottle, a mixed solvent of chloroform/acetonitrile was added to prepare a solution so that the concentration of the sample was about 1 mass%, the solution was filtered through a membrane filter, and the filtrate was subjected to LC/MS measurement under the following conditions.

### <Conditions>

Device: Liquid Chromatography-Fourier Transform Mass Spectrometer "Vanquish/Orbitrap Exploris 120" (Thermo Fischer-Tropsch Co., Ltd.)
Column: L- coulumn3 C8 (2.1 mmφ x 100 mm, 3 µ)
Eluent composition: water/methanol-based gradient conditions (ammonium acetate addition conditions)
Eluent flow rate: 0.7 mL/min
Column temperature: 40°C
Detector: FT-MS, diode array detector (DAD, 190 to 800 nm)
Sample Injection amount: 2 µL
Ionization method: ESI (m/z = 100 to 2,000) (POS)

### <Hazen Color Scale Value (APHA)>

The Hazen color scale value (APHA) of the ultraviolet light absorbing agent was measured by the method according to JIS K0071-1: 2017. A color standard solution "1000" (product name, commercially available from FUJIFILM Wako Pure Chemical Corporation) was diluted with pure water to prepare solutions of APHA, 0, 5, 10, 15, 20, and 25, and a calibration curve was created. A solution in which 3.5 g of the purified ultraviolet light absorbing agent was dissolved in 50 g of toluene was filled into a quartz cell with an optical path length of 50 mm, and the APHA value was measured using a Spectral Haze Meter "COH7700" (product name, commercially available from Nippon Denshoku Industries Co., Ltd.).

### <Luminous Transmittance>

For the luminous transmittance, the transmittance was measured by a UV-Visible/NIR Spectrophotometer "UH-4150" (product name, commercially available from Hitachi High-Tech Corporation) at the optical center of the lens substrate at a measurement wavelength of 380 to 780 nm, and the value calculated according to the International Standard ISO 8980-4, taking into account weighting by relative luminous efficiency within the wavelength range was expressed as the luminous transmittance (%).

### <L*a*b* value and YI>

The L* value, the a* value, and the b* value of the lens substrate in the L*a*b* color system were measured using a spectrophotometer U-3500 (commercially available from Hitachi, Ltd.) at the optical center of the lens substrate. The YI value wad determined according to JIS K 7373:2006.

### <Example 1>

An ultraviolet light absorbing agent containing 2-(3-tert-butyl-2-hydroxy-5-methylphenyl)-5-chloro-2H-benzotriazole was measured by the above LC/MS, and the amount of the compound (2) contained in the ultraviolet light absorbing agent was measured. As a result, the peak area ratio of the compound (2) was 0.011%. In addition, the measurement result of the APHA of the ultraviolet light absorbing agent was 295.

Then, 7.0 g of the ultraviolet light absorbing agent and 100 cc of toluene were added to a 300 cc beaker and dissolved, 50 cc of isopropanol was then added, and the mixture was uniformized at room temperature 21°C. Then, the mixture was left in a refrigerator at 5°C for 48 hours to recrystallize, and after 48 hours, the ultraviolet light absorbing agent deposited at the bottom of the beaker was removed using a Büchner funnel, and the ultraviolet light absorbing agent was washed by suction filtration using 200 cc of isopropanol cooled to 5°C. The light yellow needle-like crystal ultraviolet light absorbing agent was put into a hot air drier at 60°C and dried. Thereby, 4.0 g of the purified ultraviolet light absorbing agent was obtained. The obtained ultraviolet light absorbing agent was measured by the above LC/MS, and the amount of the compound (2) contained in the ultraviolet light absorbing agent was measured. As a result, the peak area ratio of the compound (2) was 0.0023%. In addition, the measurement result of the APHA of the purified ultraviolet light absorbing agent was 169.

### <Example 2>

0.06 parts by mass of dimethyltin dichloride as a catalyst, 0.15 parts by mass of butoxyethyl acid phosphate "JP-506H" (product name, commercially available from Johoku Chemical Co., Ltd.) as a mold releasing agent, and 1.00 part by mass of the ultraviolet light absorbing agent obtained in Example 1 were added to 50.28 parts by mass of a mixture of 2,5-bis(isocyanatomethyl)-bicyclo[2.2.1]heptane and 2,6-bis(isocyanatomethyl)-bicyclo[2.2.1]heptane, and the mixture was stirred and mixed. Then, 25.50 parts by mass of pentaerythritol tetrakis(3-mercaptopropionate), and 24.22 parts by mass of 1,2-bis(2-mercaptoethylthio)-3-mercaptopropane were additionally added, and the mixture was stirred and mixed under a reduced pressure of 10 mmHg for 30 minutes to prepare a curable composition. Next, this curable composition was injected into a lens molding mold composed of a glass mold and a resin gasket prepared in advance (0.00D, the center thickness was set to 1.6 mm) and polymerized in an electric furnace at 20°C to 120°C for 24 hours. After the polymerization was completed, the gasket and the mold were removed, and a heat treatment was then performed at 120°C for 2 hours to obtain a lens substrate. The center thickness (ct), the luminous transmittance, the L*a*b* value and the YI value were measured and shown in Table 1.

### <Comparative Example 1>

A lens substrate was obtained in the same method as in Example 2 except that the ultraviolet light absorbing agent was changed to 1.00 part by mass of an ultraviolet light absorbing agent containing 2-(3-tert-butyl-2-hydroxy-5-methylphenyl)-5-chloro-2H-benzotriazole (the peak area ratio of the compound (2) was 0.011%). The center thickness (ct), the luminous transmittance, the L*a*b* value and the YI value were measured and shown in Table 1.

### [Table 1]

**Table 1**

| | | Example 2 | Comparative Example 1 |
|---|---|---|---|
| Isocyanate component | | NBDI | NBDI |
| Active hydrogen compound component | | PETMP | PETMP |
| | | GST | GST |
| Ultraviolet light absorbing agent | Peak area ratio (%) of compound (2) | 0.0075 | 0.033 |
| | Amount (parts by mass with respect to 100 parts by mass of resin) | 1.00 | 1.00 |
| Lens substrate | ct (mm) | 1.61 | 1.63 |
| | Luminous transmittance (%) | 89.9 | 89.9 |
| | YI | 8.64 | 8.85 |
| | L* | 96.0 | 96.0 |
| | a* | -3.23 | -3.28 |
| | b* | 5.97 | 6.11 |

### <Example 3>

0.06 parts by mass of dimethyltin dichloride as a catalyst, 0.15 parts by mass of butoxyethyl acid phosphate "JP-506H" (product name, commercially available from Johoku Chemical Co., Ltd.) as a mold releasing agent, and 0.55 parts by mass of the ultraviolet light absorbing agent obtained in Example 1 were added to 50.60 parts by mass of 1,3-bis(isocyanatomethyl)benzene, and the mixture was stirred and mixed. Then, 0.55 parts by mass of 2-ethylhexyl 2-(2-hydroxy-4-ethoxyphenyl)2H-benzotriazole-5-carboxylate was additionally added and the mixture was stirred and mixed. Then, 49.40 parts by mass of a mixture of 4,7-bis(mercaptomethyl)-3,6,9-trithiaundecane-1,11-dithiol, 4,8-bis(mercaptomethyl)-3,6,9-trithiaundecane-1,11-dithiol, 5,7-bis(mercaptomethyl)-3,6,9-trithiaundecane-1,11-dithiol was additionally added, and the mixture was stirred and mixed under a reduced pressured of 10 mmHg for 30 minutes to prepare a curable composition. Next, this curable composition was injected into a lens molding mold composed of a glass mold and a resin gasket prepared in advance (0.00D, the thickness was set to 1.8 mm) and polymerized in an electric furnace at 20°C to 120°C for 24 hours. After the polymerization was completed, the gasket and the mold were removed, and a heat treatment was then performed at 120°C for 2 hours to obtain a lens substrate. The center thickness (ct), the luminous transmittance, the L*a*b* value and the YI value were measured and shown in Table 2.

### <Comparative Example 2>

A lens substrate was obtained in the same method as in Example 3 except that the ultraviolet light absorbing agent was changed to 0.55 parts by mass of an ultraviolet light absorbing agent containing 2-(3-tert-butyl-2-hydroxy-5-methylphenyl)-5-chloro-2H-benzotriazole (the peak area ratio of the compound (2) was 0.011%). The center thickness (ct), the luminous transmittance, the L*a*b* value and the YI value were measured and shown in Table 2.

### [Table 2]

**Table 2**

| | | Example 3 | Comparative Example 2 |
|---|---|---|---|
| Isocyanate component | | XDI | XDI |
| Active hydrogen compound component | | FSH | FSH |
| Ultraviolet light absorbing agent | Peak area ratio (%) of compound (2) | 0.0075 | 0.033 |
| | Amount (parts by mass with respect to 100 parts by mass of resin) | 0.55 | 0.55 |
| Lens substrate | ct (mm) | 1.87 | 1.87 |
| | Luminous transmittance (%) | 88.2 | 88.1 |
| | YI | 9.67 | 9.73 |
| | L* | 95.2 | 95.2 |
| | a* | -3.50 | -3.51 |
| | b* | 6.60 | 6.66 |

Comparing Example 2 and Comparative Example 1 and comparing Example 3 and Comparative Example 2 described above, it can be understood that, when the peak area ratio of the compound (2) is a predetermined value or less, a spectacle lens with a reduced YI value can be obtained.

## Claims

1. An ultraviolet light absorbing agent comprising a compound (1) represented by Formula (1): 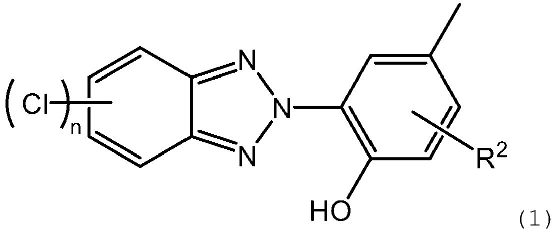 (where,
R² is an alkyl group having 1 to 12 carbon atoms or an alkoxy group having 1 to 12 carbon atoms, and
n is 0 or 1),
wherein, in a spectrum of the ultraviolet light absorbing agent measured by liquid chromatography using a diode array detector, the peak area ratio of a compound (2) represented by Formula (2) is 0.008% or less: 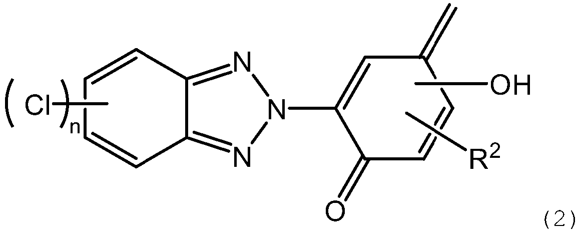 (where,
R² is an alkyl group having 1 to 12 carbon atoms or an alkoxy group having 1 to 12 carbon atoms, and
n is 0 or 1).

2. The ultraviolet light absorbing agent according to claim 1,
wherein the peak area ratio of the compound (2) in a spectrum of the ultraviolet light absorbing agent measured by liquid chromatography using a diode array detector is 0.001% or more.

3. The ultraviolet light absorbing agent according to claim 1,
wherein the peak area ratio of the compound (1) in a spectrum of the ultraviolet light absorbing agent measured by liquid chromatography using a diode array detector is 99% or more.

4. The ultraviolet light absorbing agent according to claim 1,
wherein the compound (1) is a compound (1-1) represented by Formula (1-1): 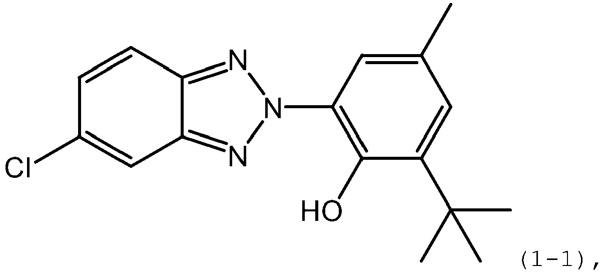 and
the compound (2) is a compound (2-1) represented by Formula (2-1):

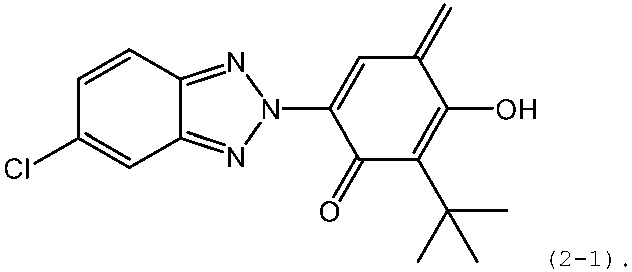

5. A spectacle lens comprising a lens substrate including the ultraviolet light absorbing agent according to any one of claims 1 to 4 and a resin.

6. The spectacle lens according to claim 5,
wherein the resin is a cured product of an isocyanate component containing at least one selected from the group consisting of bis(isocyanatomethyl)bicyclo[2.2.1]heptane, bis(isocyanatomethyl)cyclohexane, bis(isocyanatomethyl)benzene, tolylene diisocyanate, diphenylmethane diisocyanate, dicyclohexylmethane diisocyanate, hexamethylene diisocyanate, and pentamethylene diisocyanate, and an active hydrogen compound component.

7. The spectacle lens according to claim 6,
wherein the active hydrogen compound component contains at least one selected from the group consisting of toluenediamine, pentaerythritol tetrakismercaptoacetate, pentaerythritol tetrakismercaptopropionate, trimethylolpropane trismercaptoacetate, trimethylolpropane trismercaptopropionate, bis(mercaptoethylthio)mercaptopropane, bis(mercaptomethyl)-3,6,9-trithiaundecandithiol, dimercaptoethyl sulfide, bis(mercaptomethyl)dithiane, dimercaptoethyl ether and diethylene glycol.

8. The spectacle lens according to claim 7,
wherein the bis(mercaptomethyl)-3,6,9-trithiaundecandithiol is a mixture of 4,7-bis(mercaptomethyl)-3,6,9-trithiaundecane-1,11-dithiol, 4,8-bis(mercaptomethyl)-3,6,9-trithiaundecane-1,11-dithiol and 5,7-bis(mercaptomethyl)-3,6,9-trithiaundecane-1,11-dithiol.

9. The spectacle lens according to claim 5,
wherein the resin is an episulfide resin.

10. The spectacle lens according to claim 5, comprising 0.05 parts by mass or more and 2.00 parts by mass or less of the compound (1) with respect to 100 parts by mass of the resin.

11. The spectacle lens according to claim 5,
wherein the lens substrate has a refractive index ne of 1.52 or more and 1.80 or less.

12. The spectacle lens according to claim 5,
wherein the lens substrate has a refractive index ne of 1.70 or more and 1.80 or less.

13. The spectacle lens according to claim 5, comprising at least one layer selected from the group consisting of a hard coat layer, a foundation layer, and an antireflection layer.

14. A method of producing ultraviolet light absorbing agent crystals containing a compound (1) represented by Formula (1): 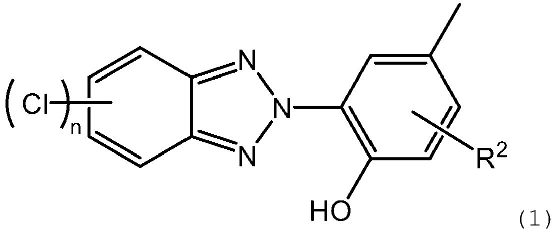 (where,
R² is an alkyl group having 1 to 12 carbon atoms or an alkoxy group having 1 to 12 carbon atoms, and
n is 0 or 1), the method comprising:
a step of adding an isopropanol solution to a toluene solution containing the ultraviolet light absorbing agent to obtain crystals; and
a step of washing the crystals with isopropanol.
